# EUROPEAN PATENT SPECIFICATION

(11) **EP 2 491 960 B1**
(45) Date of publication and mention of the grant of the patent: **15.03.2017**
(21) Application number: 09850624.9
(22) Date of filing: 03.12.2009
(51) Int. Cl.: A61L 27/26

(54) **COMPOSITION FOR CARTILAGINOUS TISSUE REPAIR AND A PRODUCTION METHOD THEREFOR**
ZUSAMMENSETZUNG ZUR KNORPELGEWEBEREPARATUR UND HERSTELLUNGSVERFAHREN DAFÜR
COMPOSITION POUR REPARER UN TISSU CARTILAGINEUX ET SON PROCEDE DE PREPARATION

(30) Priority: 23.10.2009 KR 20090101388
(43) Date of publication of application: 29.08.2012
(73) Proprietor: Sewon Cellontech Co., Ltd, Seoul (KR)
(72) Inventor: JANG, Cheong-Ho, Seoul (KR); YU, Ji-Chul, Seoul 156-718 (KR); LEE, Sae-Bom, Seoul 138-891 (KR); PARK, Hyun-Shin, Seoul 158-076 (KR); KIM, Hyun-jo, Seoul 136-827 (KR); JANG, Jae-Deog, Seoul 139-784 (KR); YEO, Se-Ken, Yongin-si Giheung-gu Gyeonggi-do 446-740 (KR); PARK, Ju-Hee, Seoul 142-060 (KR); KIM, Seok-Jung, Seoul 137-040 (KR)
(74) Representative: dompatent von Kreisler Selting Werner - Partnerschaft von Patent- und Rechtsanwälten mbB
(86) International application number: PCT/KR2009/007188
(87) International publication number: WO 2011/049265

(56) References cited:
- WO-A1-2005/060987
- WO-A2-01/37889
- WO-A2-2006/072622
- KR-A- 20090 095 647
- KR-A- 20090 101 273
- US-A1- 2007 134 291

## Description

### Field of the Invention

The present invention relates to a composition for cartilaginous tissue repair and a production method thereof, more precisely, biomaterials such as collagen and fibrin are mixed so as to allow damaged cartilaginous tissue to be repaired to a state allowing transplantation onto the tissue, and efficient regeneration is induced, thereby making it possible to reduce surgery-related stress on people and animals while inducing relatively rapid and efficient cartilage repair and regeneration, and, as a result, making it possible to substantially improve product quality and reliability and give a good impression to patients.

### Description of the Related Art

The articular cartilage found on many joint surfaces is an extremely slippery and shiny material which functions to reduce friction and wear resistance of joints during actions of joints. Articular cartilage friction is increased when the articular cartilage is damaged, and the articular cartilages becomes wear out and erode, and pain and functional disorder occur, and cartilage damage on the joint becomes osteoarthritis. The causes of the cartilage damage are trauma such as fall, direct hit or turning force, or diseases such as arthritis, osteonecrosis, inflammatory arthritis or osteochondritis dissecans, and symptoms are pain, edema, and catching. Many studies for the physiological regeneration of the articular cartilage are carried out for tens of years to treat articular cartilage damage and several treatments are used.

However, according to the reports, until now, a common treatment such as a physical therapy or a drug treatment for the treatment of articular cartilage damage is used because the articular cartilage tissues cannot be regenerated. Operative and non-operative treatments are used. Hot and cold packs and medication such as NSAID, and intraarticular injection of steroids are examples of the non-operative treatment which only palliates the symptoms and does not weaken the disease. Reparative procedures such as debriment, microfracture, drilling/abrasion arthroplasty, autologous osteochondroal and transplantation, chondrocyte implantation, and articular cartilage replacement are operative treatment.

Recently, there are several attempts of an operative treatment that biomaterials are applied onto the cartilage defect region.

Biomaterials are compatible with a human body and do not show any rejection. These biomaterials can replace or regenerate the damaged tissue and organs to the normal tissue and normal organs. Therefore, attentions about the biomaterials which can be used to replace or regenerate the damaged tissue and organs are increased.

Human body rejects foreign material in his body. Therefore, substitution of a part of a human organ with other materials is very difficult. The biomaterials contribute to the progress of medical surgery because the biomaterials have biological affinity for human body.

There are two types of biomaterials, synthetic and natural biomaterials which can be used to manufacture implantation medical devices for diagnosis and treatment.

Metals, Inorganic materials, ceramics, synthetic polymers belong to synthetic biomaterials that show no rejection but do not have vitality.

Natural biomaterials such as fibrin, collagen, hyaluronic acid, and chitosan are developed and commercialized.

The materials which form human body and sustain life are biological polymers and cells. Polysaccharides and proteins found in human body are representative examples. If they have no immune rejection, they can be a state similar to a natural state and expected to regeneration of human organs which have growth function. Natural tissues can be used for a medical biomaterial which can give biological functions to inorganic and synthetic polymeric materials. Biocompatibility between surrounding tissues and inorganic biomaterials transplanted in human body can be established by the natural tissues, and further, the natural tissues can give biological functions to inorganic biomaterials.

Biocompatible and biodegradable fibrin is used as a natural adhesive and an antihemorrhagic agent. Fibrin is absorbed in several weeks during wound healing. It has been reported that fibrin has no side effects such as inflammation, immune response, parenchyma necrosis, and fiber hypertrophy.

Fibrin having a form of natural supporter for fibroblast cell has an important role in wound healing.

The conception of fibrin products was established in 1970s and the first industrial fibrin product was sold at Europe in 1982 and has been sold until now. Recent studies proved that fibrin can be used as a supporter for tissue engineering, and optimization is carried out in several medical fields such as orthopedics, the dentist, and neurosurgery.

Collagen is a group of proteins and is found on dermis, tendon/ligament, blood vessel, bone, and cartilage. One third of total protein in mammals is collagen.

More than 20 different types of collagen are reported, among them the amount of collagen type I which is found in skin, tendon/ligament, and bone is about 90 % of total collagen.

Collagen is composed of a triple helix, whose molecular weight is 300,000 dalton (100,000 dalton each helix). The smallest amino acid glycine is found at every third position (-GXY-; X and Y are any amino acids) of collagen molecule. Thus, total glycine in collagen is one third of the total amino acids. Collagen has hydroxyproline and the content of hydroxyproline is 10 % of total amino acids. Hydroxyproline is used for the quantitative analysis of collagen.

Collagen is used in several medical fields, such as a styptic, a wound dressing, artificial vascular, and agents for wrinkle improvement. The first collagen styptic Aviten which has powder form extracted from calf skin is developed in 1974 and is used until now.

There are 3 ways of the use of collagen as a raw material. Pure collagen, processed collagen which is processed through acellular/decellular process of tissue, and altered collagen are used.

Pure collagen has low tensile strength, thus it is not recommended to be used for suture in spite of its high stability and purity. Collagen has lowered tensile and tear strength than other polymers, so that other materials such as GAG or biocompatible synthetic polymers (PGA/PLA) are added to collagen to improve their strengths.

Collagen has many advantages such as low antigenicity, high biocompatibility, and bioabsorbablity, adhesion of cells, cell growth, cell differentiation induction, blood coagulation, styptic effect, and biocompatibility with other polymers.

However, physical properties and characteristics for maintaining volume are inferior and pure collagen is expensive.

Fibrin has moderate properties in volume, elasticity, and adhesiveness, and also has styptic effect. Thus, fibrin is an effective biomaterial for the cartilaginous tissue repair and regeneration.

Articular cartilage is avascular and non-nerve tissue, and has very limited self healing ability unlike the other mesenchymal tissue. Thus, one of the objects of the present invention is to provide nutrients which are used for cartilaginous tissue regeneration by the addition of medium components for cultivation of chondrocyte.

When cartilage tissue of a joint is once damaged, it cannot be normally regenerated in body. The patient undergoes limited daily life with severe pain, and when it becomes chronic it induces fatal osteoarthritis that makes normal life of the patient becomes impossible. There are more than 500,000 cases of arthroplasty and the entire joint replacement surgery in USA and Europe, respectively.

Thereby, a simple procedure for the cartilage defect region treatment by the use or transplantation of biomaterials such as collagen and fibrin is required. These cartilage defect region treatment methods are very effective in the early stage of cartilage damage. And when such treatment is carried out in the early stage of cartilage damage, the number of patients who need knee replacement surgery can be lowered. By this preventive treatment the number of osteoarthritis will also be lowered.

In the past, it was not reported that composition for cartilaginous tissue repair by the use of biomaterials such as collagen and fibrin glue and the methods to apply them for treatment.
WO 2005/060987 A1 is directed to a cartilage therapeutic composition comprises a mixture of chondrocytes, and thrombin and a fibrinogen matrix containing fibrinogen.
WO 01/37889 A2 is directed to a bone-substitute material, comprising a soft matrix, living cells and a curing matrix.

### Summary of the Invention

The present invention is provided to solve the problems of the prior art. The first object of the present invention is to provide a composition for cartilaginous repair composed of fibrinogen and aprotinin solution, thrombin and a stabilizing solution, and a collagen solution. The second is effective regeneration of damaged cartilaginous tissue by a transplantable form composed of biomaterials such as collagen and fibrinogen. This treatment reduce burden of surgery, and regeneration and restoration of cartilage defect region is induced. The third is that this method makes simple treatment of cartilage defect region possible. The fourth is reduction of the number of patients who require joint surgery by the early treatment of the cartilage defect region. The fifth is reduction of the number of patients who require joint surgery by the preventive treatment. The sixth is to provide a composition the cartilaginous tissue repair and method of making the composition, so that improved quality and reliability of the product imparts good image to the patients.

For the achievement of the objectives, the present invention provides a method making a composition for cartilaginous tissue repair comprising the steps of (a) dissolving freeze-dried fibrinogen in an aprotinin solution; (b) dissolving freeze-dried thrombin in a stabilizing solution; (c) mixing an enriched collagen solution with thrombin and the stabilizing solution; and installing the fibrinogen solution (a) to one side of a dual kit and the solution (c) containing the collagen solution to the other side, and then mixing (a) and (c), wherein the gelation time after mixing (a) and (c) is 3 minutes and the maximum stress is above 10 N, as measured with a Rheo meter CR-500DX, wherein the concentration of fibrinogen ranges from 35 to 55 mg/L, the concentration of aprotinin solution is 1,500 KIU/mL, the concentration of thrombin is 29.41 IU/mL, the concentration of the stabilizing solution is 0.65 mg/mL, and the concentration of collagen is 13.23 mg/mL, wherein the stabilizing solution is prepared by the addition of calcium chloride to the DMEM culture medium for the cartilage cell culture, and the DMEM culture medium comprises salts, amino acids, and vitamins.

The present invention provides effective regeneration of damaged cartilaginous tissue by a transplantable form composed of biomaterials such as collagen and fibrinogen, and this method reduces burden of surgery, and rapid and effective regeneration and restoration of cartilage defect region is induced.

The present invention provides simple method for treatment of cartilage defect region.

The present invention provides reduction of the number of patients who require joint surgery by the early treatment of the cartilage defect region.

Further, the present invention provides reduction of the number of patients who require joint surgery by the preventive treatment.

The present invention provides improved quality and reliability of the product that imparts good image to the patients.

### Brief Description of the Drawings

FIG. 1 shows a production method of a composition for cartilaginous tissue repair according to the present invention.
FIG. 2 shows a photograph of application of the dual kit containing the composition for cartilaginous tissue repair according to the present invention.
FIG. 3 shows a photograph of application of the composition for cartilaginous tissue repair according to the present invention to a cartilage defect region of an animal (pig).
FIG. 4 shows photographs that show visual observations of experimental results of an application of the composition for cartilaginous tissue repair according to the present invention to a cartilage defect region of an animal (rabbit).
FIG. 5 shows photographs that show histopathological observations of experimental results of an application of the composition for cartilaginous tissue repair according to the present invention to a cartilage defect region of an animal (rabbit).
FIG. 6A and 6B show photographs of gelation of a solution according to the present invention and the prior art, respectively.
FIG. 7A and 7B show fluorescence microscope photographs of cartilage cells viability.

### Detailed Description of the Invention

Hereinafter, embodiments of the present invention will be described in detail with attached Figs.

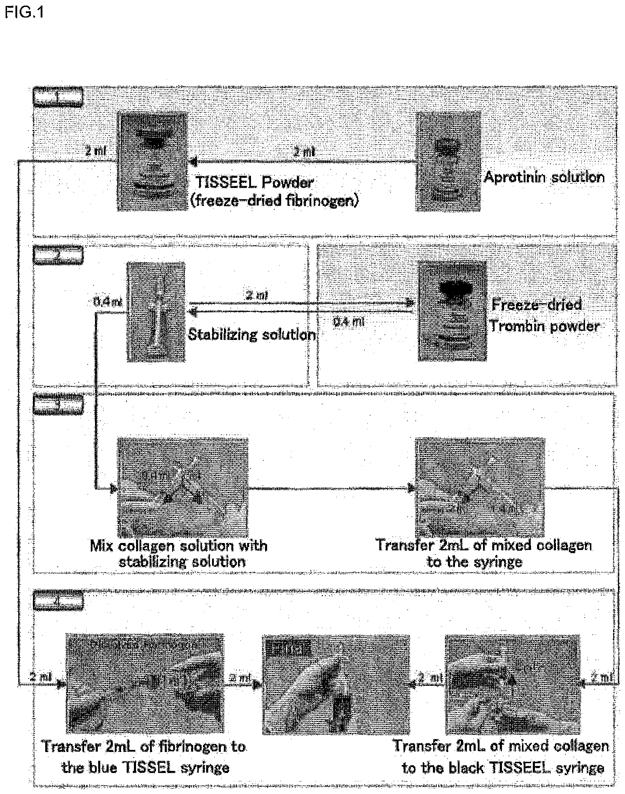

A composition for cartilaginous tissue repair and a method of making the composition according to the present invention, and applications of them are shown in FIGS. 1-5.

When a detail explanation regarding related known art and technical configuration unnecessarily confuses gist of the present invention, the detail explanation is omitted.

And the definitions in the present invention are to be interpreted from the contents of the present invention because the terms may be interpreted by the intention of the producer or custom.

First of all, the present invention provides solid material using biomaterials such as collagen and fibrinogen, and also provides making and usage of a stabilizing solution which gives nutrition to an environment for the cartilage regeneration, and then a composition for cartilaginous tissue repair which is made through following several steps is provided.

For details, the steps for making a composition for cartilaginous tissue repair are composed of (a) dissolving freeze-dried fibrinogen in an aprotinin solution;
(b) dissolving freeze-dried thrombin in a stabilizing solution;
(c) mixing an enriched collagen solution with thrombin and the stabilizing solution;
and installing the fibrinogen solution (a) to one side of a dual kit and the solution (c) containing the collagen to the other side, and then mixing (a) and (c),
wherein the gelation time after mixing (a) and (c) is 3 minutes and the maximum stress is above 10 N, as measured with a Rheo meter CR-500DX,
wherein the concentration of fibrinogen ranges from 35 to 55 mg/L, the concentration of aprotinin solution is 1,500 KIU/mL, the concentration of thrombin is 29.41 IU/mL, the concentration of the stabilizing solution is 0.65 mg/mL, and the concentration of collagen is 13.23 mg/mL,
wherein the stabilizing solution is prepared by the addition of calcium chloride to the DMEM culture medium for the cartilage cell culture, and the DMEM culture medium comprises salts, amino acids, and vitamins, and injecting into damaged cartilaginous tissue.

A 0.26 mg/mL of calcium chloride is contained in the stabilization solution.

The final calcium chloride concentration in the stabilizing solution ranges from 0.1 to 0.5 mg/mL.

And the final calcium chloride concentration in the composition for cartilaginous tissue repair ranges from 2.78 to 3.12 mg/mL.

The production of the stabilizing solution in the present invention will be explained in detail as follows.
- Calcium chloride is added to the DMEM culture medium used in culture of chondrocyte to make the stabilizing solution. The DMEM culture medium contains salts, amino acids, and vitamins. The calcium chloride concentration ranges from 0.2 to 6 mg/mL.
- The final calcium chloride concentration in the collagen and the stabilizing solution ranges from 0.1 to 0.5 mg/mL.
- The calcium chloride concentration in the stabilizing solution is determined by gelation time and the maximum stress range. The gelation time is 3 minutes and the maximum stress is above 10N.
- The minimum range of the stabilizing solution is the minimum range used for the culture condition, and 0.5 mg/mL of calcium chloride is the proved maximum range for the stabilization. The final calcium chloride concentration used in the commercial fibrin glue ranges from 2.78 to 3.12 mg/mL which affects the cells.

The concentrated collagen is prepared as follows;
Highly enriched collagen is preferable to use.

Collagen (under 5mg/mL) is sterilized by using 0.22 um filter and then concentrated under aseptic manipulation.

The molecular weight of collagen is about 300,000 Dalton, and the length of the collagen molecule is about 300 nm so that filtration is difficult when the concentration of collagen is higher than 5 mg/mL.

Collagen is concentrated in the range of 5 to 100 mg/mL.

The collagen concentration begins at 5 mg/L and it is possible to continue to 100 mg/mL (soluble and measurable concentration).

Dialysis and diafiltration is used for collagen concentration, or centrifugal method under certain pH and temperature is used. The concentrated collagen is placed in a syringe for further use.

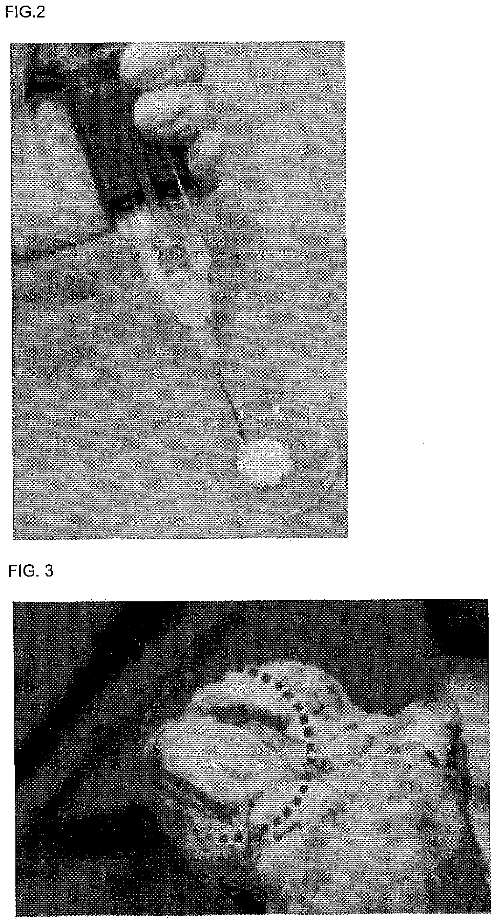

The mixing of collagen, fibrinogen, and the stabilizing solution is illustrated in the FIG. 2.
- Dissolve freeze-dried fibrinogen contained in a fibrin glue product in 2 cc aprotinin solution and then the solution is placed on a syringe.
- Dissolve freeze-dried thrombin in the 2 cc stabilizing solution and then the solution is placed on a 0.4 cc syringe.
- Concentrated collagen (3 %, 3 cc) solution is mixed with the thrombin solution, and then the solution is placed on a 2 cc syringe.
- The fibrinogen/aprotinin solution and the collagen/thrombin solution are installed in a dual kit and then are applied to the cartilage defect region.
- There are various fibrinogen (fibrin glue) products sold in several countries, and in products used in Korea are listed the table 1.

**Table 1.**

| | | | product Name | | |
|---|---|---|---|---|---|
| | | | GreenPlast | Beriplast | Tissucol/ Tisseel |
| manufacture | | | Green Cross | ZLB Behring GmbH | Baxter AG |
| Country | | | R. of Korea | Germany | Austria |
| coagulation factor | human plasma fibrinogen | mg/mL | 71.5∼126.5 | 65∼115 | 70∼110 |
| | human plasma thrombin | IU/mL | 400∼600 | 400∼600 | 500 |
| | human plasma Factor XIII | U/mL | 44∼88 | 40∼80 | 10∼50 |
| coagulation catalyst | calcium chloride | mg/mL | 5.56-6.24 | 5.9 | 5.88 |
| thrombolysis inhibitor | aprotinin | KIU/mL | 1000 | 1000 | 3000 |

Hemassel (Canada), Quixil (Israel), Bolheal (Japan), Biocol (France), and Vanguard (USA) are also available in other countries.

Embodiments of a composition for cartilaginous tissue repair and a production method thereof are explained as follows.

First of all, the present invention provides biomaterials such as collagen and fibrin are mixed so as to allow damaged cartilaginous tissue to be repaired to a state allowing transplantation onto the tissue, and efficient regeneration is induced, thereby making it possible to reduce surgery-related stress on people and animals while inducing relatively rapid and efficient cartilage repair and regeneration. Hereinafter, embodiments of the present invention will be described in detail.

### Embodiment 1

A method to apply collagen and fibrin glue to a cartilage defect region of an animal (object: confirm the possibility of an application of collagen and fibrin glue to a cartilage defect region of a pig).
- After a pig leg is fixed on a mount, cartilage is damaged by a drill (2 x 1.5 cm²).
- Thera Fill (enriched collagen) and Greenplast (fibrin glue) are filled in the cartilage defect region. The filling method is as follow.
   1) Open the Greenplast and then add fibrinogen to an aprotinin solution by injection.
   2) add a freeze dried thrombin to a calcium solution.
   3) A 0.1 mL of thrombin/calcium solution is mixed with 1 cc of Thera Fill by using an adapter.
   4) The solution 1) and 3) are installed in a dual kit and then apply them to the cartilage defect region.

After the application, observe the cartilage defect region for 15 minutes.

(Result: after the application of Thera Fill/ fibrin glue to the cartilage defect region, solid materials are formed within 10 minutes and the solid materials have good characteristics for practitioners. Refer to FIG. 3)

### Embodiment 2

An application experiment of collagen and fibrin glue to a cartilage defect region of a rabbit (object: confirm the regeneration of cartilage of the rabbit by using collagen and fibrin glue).

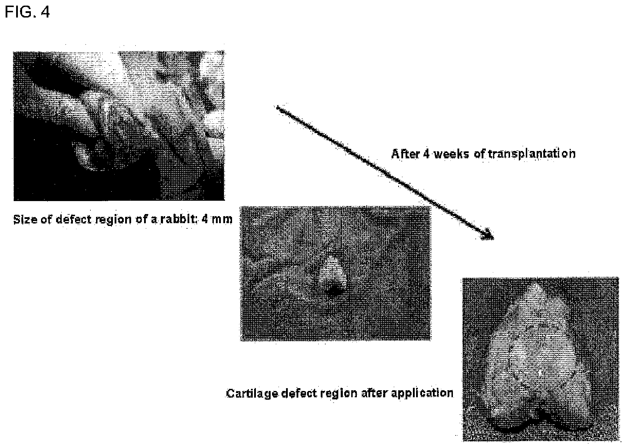

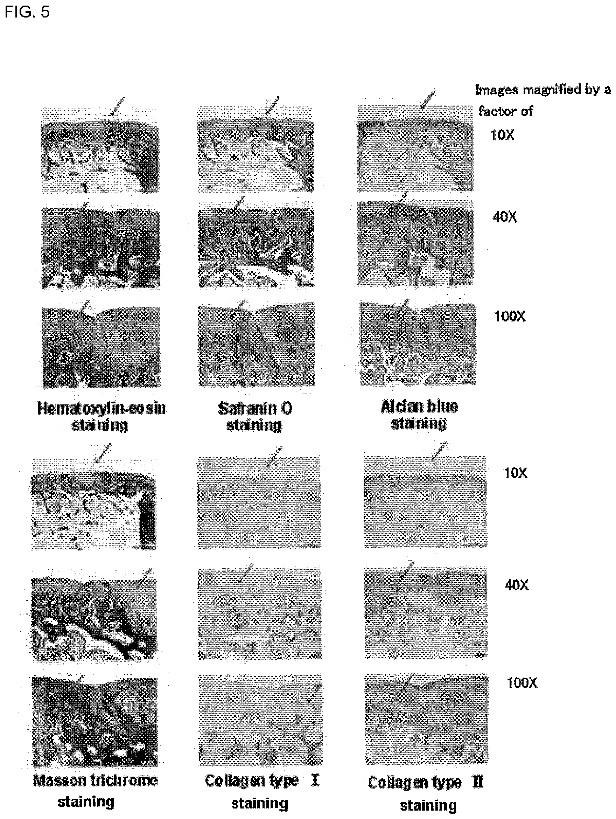

After a 4 mm of cartilage defect is induced in the patellar groove region of a NZW rabbit, collagen and fibrin glue are applied to the defect region, and then after 4 weeks of the application, naked eye and histopathology observations (FIGS. 4 AND 5) are carried out. Collagen (Thera Fill from sewoncellontech) and Fibrin (Tisseel from Baxter) are used for the experiment.

Hematoxylin-eosin, Safranin-O, Alcian-blue, Masson's trichrome, and Collagen type I/II are used for staining for histopathology observations.

(Result: it was confirmed by the naked eye observation that the defect region tissues are restored, and by histopathology observation it was confirmed that the defect region tissues are regenerated as cartilage tissues.)

The physical properties of the stabilizing solution depending on the calcium chloride concentration are measured and summarized as follows
A. Measurement of gelation time and physical properties according to the calcium chloride concentration in the stabilizing solution.

### 1) Calcium chloride concentration

| | **Final concentration (mg/mL)** | | | | | |
|---|---|---|---|---|---|---|
| | 0.26 | 0.20 | 0.10 | 0.05 | 0.01 | 0 |
| Concentration of the stabilizing solution | 4.4 | 3.38 | 1.69 | 0.845 | 0.169 | 0 |

### 2) Application method

- Prepare a collagen solution (3 %, 3 cc) and a fibrin glue (from Greenplast).
- Fibrinogen in Greenplast is mixed with an aprotinin (1 cc).
- Thrombin is mixed with the stabilizing solution (1cc).
- Collagen solution (3 cc) is mixed with Thrombin (3 cc) and the stabilizing solution (0.4 cc).
- Place the fibrinogen solution (1 cc) and collagen solution (1 cc) in a dual kit and then put the mixture into a cylindrical mold (∅ 12 X 15 mm) to make a solid matter.

### 3) Calcium chloride concentration/ gel time and gelation.

| Concentration (mg/mL) | Gel time (min) / Gelation | | | | |
|---|---|---|---|---|---|
| | 0.5 | 1 | 1.5 | 2 | 3 |
| 0.26 | * | * | ** | ** | *** |
| 0.2 | * | * | ** | *** | *** |
| 0.1 | * | * | ** | ** | *** |
| 0.05 | * | * | * | * | ** |
| 0.01 | * | * | * | * | ** |
| 0 | * | * | * | * | ** |

| | | | | | |
|---|---|---|---|---|---|
| *: includes liquid phase **: in progress of gelation ***: gelation completed | | | | | |

Measurement of physical properties depending on the calcium chloride concentration.
- Rheo meter, CR-500DX, is used to measure the physical properties of the solid material.
- Measurement items: the maximum stress, the gel strength, and the tensile strength.
- Entry distance: 50 % of the sample height, 7.5 mm.
- Speed: 50 mm/min, maximum stress: 10 kg.
- Adapter: No.1, ∅ 20 mm.

| Calcium chloride concentration (mg/mL) (concentration in the final product) | Maximum stress (Max 1) | Gel strength | Tensile strength |
|---|---|---|---|
| | N | g.cm | g/cm |
| 0.26 | 19.3 | 1212.9 | 2493.7 |
| 0.2 | 20.1 | 1246.6 | 2591.1 |
| 0.1 | 14.9 | 918.8 | 1920.3 |
| 0.05 | 6.1 | 303.1 | 789.9 |
| 0.01 | 6.9 | 366.7 | 888.6 |
| 0 | 5.4 | 281.6 | 691.1 |

Method of mixing of collagen and fibrin glue with the stabilizing solution in the cartilage defect region, and measurement of physical properties, and confirmation of degradability (object: nutrition is blocked when cartilages are once damaged. DMEM culture medium which is used for culturing cartilage cells is added to help restoration of the cartilage, and the product containing collagen maintains the shape for a long time).
1) Making a stabilizing solution that is a calcium chloride strengthened DMEM culture medium which is used for culturing cartilage cells. The DMEM culture medium contains salts, amino acids, and vitamins. The stabilizing solution is a calcium chloride strengthened solution, and the concentration ranges from 0.2 to 6 mg/mL.
2) The calcium chloride concentration ranges from 0.1 to 0.5 mg/mL in the final use.
3) This experiment is carried out as follows.
- Prepare collagen solution (3 %, 3 cc), and fibrin glue (Greenplast)
- Fibrinogen in Greenplast is mixed with an aprotinin (1 cc).
- Thrombin is mixed with the stabilizing solution (1 cc).
- Collagen solution (3 cc) is mixed with thrombin/stabilizing solution (0.4 cc).
- Fibrin glue not containing collagen is used for a comparison group.
- Place the fibrinogen solution (1 cc) and collagen solution (1 cc) in a dual kit and then put the mixture into a cylindrical mold (∅ 12 X 15 mm) to make a solid matter. The final calcium chloride concentration is 0.26 mg/mL.

4) Measurement of physical properties
- Rheo meter, CR-500DX, was used to measure the physical properties of the solid material.
- Measurement items: the maximum stress, the gel strength, and the tensile strength.
- Entry distance: 50 % of the sample height, 75 mm.
- Speed: 50 mm/min, maximum stress: 10 kg.
- Adapter: No.1, ∅ 20 mm.
- Results are as follows. A solid material containing collagen shows opaque color similar to the cartilage color, and a fibrin glue product is a translucent solid material.

| | Collagen/stabilizing solution (the present invention) | Fibrin glue product (prior art) |
|---|---|---|
| Photos of gelation (gelation in 3 minutes) | FIG. 6A | FIG. 6B |

5) Confirmation of degradability
- The solid material is placed in the culture medium (DMEM) and is observed for one month at 37 degree Celsius.
- The culture medium is changed everyday or every 2 days.
- Fibrin glue product not containing collage is decomposed in 2 to 3 weeks but the solid material containing collagen maintains its shape over one month.

Next is an experiment about cell viability of cartilage cells in the composition of collagen and fibrinogen mixture.
A) Preparation of a composition of collagen and fibrinogen containing cartilage cells
   1) Fibrinogen in fibrin glue (Tisseel) is dissolved in an aprotinin solution (2cc).
   2) A freeze-dried thrombin is dissolved in the stabilizing solution.
   3) Collagen solution (Thera fill, 3 cc) is mixed with the thrombin/stabilizing solution (0.4 cc) by the use of an adapter.
   4) Put a 1.5 cc of the mixed collagen/thrombin/the stabilizing solution in a syringe, and then the solution is mixed with the cartilage cells (0.5 cc (6.0 X 10⁶ cells/0.5 cc)). The final calcium chloride concentration is 0.24 mg/mL.
   5) The mixture 1) and 4) are respectively installed in a dual kit, and then place them on a 100 mm petri dish.
B) A confirmation experiment of the cartilage cells viability in the composition.
   1) The detached gel product is transferred to a 35 mm petri dish, and 3 mL of DMEM culture medium is added to the petri dish, and then 37 degree Celsius CO₂ incubation is in progress.
   2) The culture medium is exchanged two times a day, and incubated 3 days. The culture medium composition is composed of DMEM/F12, 1 % gentamycin, 5 mg/mL ITS+Premix, 50 ug/mL ascorbic acid, 1mM sodium pyruvate, and 100 ng/mL BMP-2.
   3) Calcein-AM method is used for an analysis.

### Calcein-AM analysis

- Method: A Live/Dead cell viability working solution containing 10 mL PBS, 5 uL 4mM calcein-AM and 20 uL 2mM EthD-1 is prepared.

The culture medium is removed from the incubating composition, and then 3 mL of Live/Dead cell viability working solution is added. It is incubated 1 hour at room temperature, and then it is moved to a glass slide. It is observed in fluorescence microscope.

The living cells are stained in green, and the dead cells are stained in red.
- Result: Fluorescence microscope photographs of cartilage cells viability by Calcein-AM analysis.

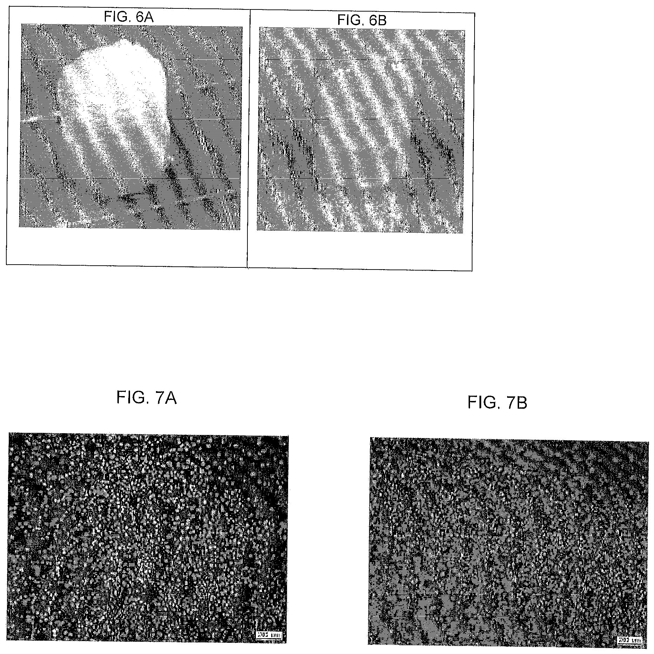

The fluorescence microscope photographs are shown in FIG. 7A (Initial (40X)) and FIG. 7B (3 day incubation (40X)).

After 72 hours in the composition, the observation confirms that 90 % of the cells are alive.

## Claims

1. A production method of a composition for cartilaginous tissue repair, comprising the steps of (a) dissolving freeze-dried fibrinogen in an aprotinin solution; (b) dissolving freeze-dried thrombin in a stabilizing solution; (c) mixing an enriched collagen solution with thrombin and the stabilizing solution; and installing the fibrinogen solution (a) to one side of a dual kit and the solution (c) containing the collagen to the other side, and then mixing (a) and (c), wherein the gelation time after mixing (a) and (c) is 3 minutes and the maximum stress is above 10 N, as measured with a Rheo meter CR-500DX,
wherein the concentration of fibrinogen ranges from 35 to 55 mg/L, the concentration of aprotinin solution is 1,500 KIU/mL, the concentration of thrombin is 29.41 IU/mL, the concentration of the stabilizing solution is 0.65 mg/mL, and the concentration of collagen is 13.23 mg/mL,
wherein the stabilizing solution is prepared by the addition of calcium chloride to the DMEM culture medium for the cartilage cell culture, and the DMEM culture medium comprises salts, amino acids, and vitamins.

## Patentansprüche

1. Herstellungsverfahren für eine Zusammensetzung zur Reparatur von Knorpelgewebe, umfassend die Schritte: (a) Auflösen von gefriergetrocknetem Fibrinogen in einer Aprotininlösung; (b) Auflösen von gefriergetrocknetem Thrombin in einer stabilisierenden Lösung; (c) Mischen einer angereicherten Collagenlösung mit Thrombin und der stabilisierenden Lösung; und Einfüllen der Fibrinogenlösung (a) auf einer Seite einer Doppelkammerspritze und der Lösung (c), die das Collagen enthält, auf der anderen Seite und dann Mischen von (a) und (c), wobei die Gelierungszeit nach dem Mischen von (a) und (c) 3 Minuten beträgt und die maximale, mit einem Rheometer CR-500DX gemessene Spannung über 10 N beträgt;
wobei die Konzentration von Fibrinogen im Bereich von 35 bis 55 mg/l liegt, die Konzentration der Aprotininlösung 1500 KIU/ml beträgt, die Konzentration von Thrombin 29,41 IE/ml beträgt, die Konzentration der stabilisierenden Lösung 0,65 mg/ml beträgt und die Konzentration von Collagen 13,23 mg/ml beträgt;
wobei die stabilisierende Lösung durch die Zugabe von Calciumchlorid zu dem DMEM-Kulturmedium für die Knorpelzellkultur hergestellt wird und das DMEM-Kulturmedium Salze, Aminosäuren und Vitamine umfasst.

## Revendications

1. Procédé de préparation d'une composition pour la réparation des tissus cartilagineux, comprenant les étapes consistant à : (a) dissoudre du fibrinogène lyophilisé dans une solution d'aprotinine, (b) dissoudre de la thrombine lyophilisée dans une solution stabilisante, (c) mélanger une solution enrichie de collagène avec la thrombine et la solution stabilisante, et placer la solution de fibrinogène (a) sur un côté d'une seringue à double cylindre et la solution (c) contenant la collagène sur l'autre côté, et ensuite mélanger (a) et (c), dans lequel le temps de gélation après le mélange de (a) et (c) est 3 minutes, et la contrainte maximale est supérieure à 10 N, telle que mesurée avec un rhéomètre CR-500DX,
dans lequel la concentration de fibrinogène est comprise entre 35 et 55 mg/l, la concentration de la solution d'aprotinine est de 1500 UIK/ml, la concentration de thrombine est de 29,41 UI/ml, la concentration de la solution stabilisante est de 0,65 mg/ml, et la concentration de collagène est de 13,23 mg/ml,
dans lequel la solution stabilisante est préparée par l'addition de chlorure de calcium au milieu de culture DMEM pour la culture de cellules cartilagineuses, et le milieu de culture DMEM comprend des sels, des acides aminés et des vitamines.
